# EUROPEAN PATENT SPECIFICATION

(11) **EP 2 560 997 B1**
(45) Date of publication and mention of the grant of the patent: **20.12.2017**
(21) Application number: 11771427.9
(22) Date of filing: 29.03.2011
(51) Int. Cl.: C08F 2/06, C08F 10/02, C08F 4/69

(54) **BY-PRODUCT MITIGATION IN AN ETHYLENE OLIGOMERISATION PROCESS**
NEBENPRODUKTUNTERDRÜCKUNG IN EINEM ETHYLENOLIGOMERISIERUNGSVERFAHREN
RÉDUCTION DES SOUS-PRODUITS DANS UN PROCÉDÉ D'OLIGOMÉRISATION DE L'ÉTHYLÈNE

(30) Priority: 20.04.2010 CA 2701252
(43) Date of publication of application: 27.02.2013
(73) Proprietor: Nova Chemicals (International) S.A., 1700 Fribourg (CH)
(72) Inventor: JABER, Isam, Calgary Alberta T2E 4T5 (CA); CARTER, Charles Ashton Garret, Calgary Alberta T3A 6L7 (CA); CLAVELLE, Eric, Calgary Alberta T2K 2N9 (CA); KRZYWICKI, Andrzej, Calgary Alberta T2N 3N9 (CA); JOBE, Ian Ronald, Calgary Alberta T2Y 2T9 (CA); CHISHOLM, P., Scott, Calgary Alberta T2L 1S8 (CA); SERHAL, Kamal Elias, Calgary Alberta T3B 4K2 (CA); ARCHER, Russell Kirk, Airdrie Alberta T4B 2P5 (CA); BESENSKI, Keith Wayne, Calgary Alberta T3B 5W1 (CA)
(74) Representative: Watson, Robert James
(86) International application number: PCT/CA2011/000318
(87) International publication number: WO 2011/130822

(56) References cited:
- US-A1- 2007 043 181
- US-A1- 2008 242 811
- US-B2- 7 022 788

## Description

### TECHNICAL FIELD

This invention provides a means to mitigate polymer formation during an ethylene oligomerization process.

### BACKGROUND ART

Alpha olefins are commercially produced by the oligomerization of ethylene in the presence of a simple alkyl aluminum catalyst (in the so called "chain growth" process) or alternatively, in the presence of an organometallic nickel catalyst (in the so called Shell Higher Olefins, or "SHOP" process). Both of these processes typically produce a crude oligomer product having a broad distribution of alpha olefins with an even number of carbon atoms (i.e. butene-1, hexene-1, octene-1 etc.). The various alpha olefins in the crude oligomer product are then typically separated in a series of distillation columns. Butene-1 is generally the least valuable of these olefins as it is also produced in large quantities as a by-product in various cracking and refining processes. Hexene-1 and octene-1 often command comparatively high prices because these olefins are in high demand as comonomers for linear low density polyethylene (LLDPE).

Technology for the selective trimerization of ethylene to hexene-1 has been recently put into commercial use in response to the demand for hexene-1. The patent literature discloses catalysts which comprise a chromium source and a pyrrolide ligand as being useful for this process - see, for example, United States Patent ("USP") 5,198,563 (Reagen et al., assigned to Phillips Petroleum).

A selective oligomerization process in which ethylene is trimerized to hexane-1 is disclosed in U.S. patent 4,668,838 (Briggs). The Briggs process uses a chromium catalyst and a cocatalyst that is prepared by the partial hydrolysis of an aluminum alkyl. The terms "aluminoxane" and "alumoxane" are now in common use to refer to partially hydrolyzed aluminum alkyls (and these terms are used as such herein). The chromium catalysts disclosed by Briggs include a heteroligand which is preferably an isonitrile, amine or ether. One potential problem with the Briggs process is that it can produce significant quantities of polymer (as disclosed in the examples of the Briggs patent).

The Briggs patent refers to an earlier patent by Manyik et al. (U.S. 3,347,840). The Manyik patent teaches the polymerization of ethylene using a chromium catalyst on an aluminoxane cocatalyst - but, as noted in the Briggs patent described above, the Manyik process also produces a significant amount of hexene as a by-product. Thus, as a quick summary: the Manyik et al. process produces polyethylene (with hexene as a by-product) and the Briggs process produces hexene (with a polyethylene by-product).

U.S. 6,800,702 (Wass) teaches the selective trimerization of ethylene using a Cr catalyst on an aluminoxane cocatalyst, where the catalyst contains a polydentate heteroatom ligand. As use herein, the term "polydendate" refers to a ligand that coordinates to the transition metal (Cr) with at least two bonds.

Preferred Wass ligands include those described by the following formula:

(X₁)(X₂)P₁ - bridge - P₂(X₃)(X₄)

in which X₁, X₂, X₃ and X₄ are most preferably phenyl groups having a polar substituent and the bridge is an amine group (e.g. R where R is a C₁ to C₄ alkyl). 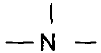 The use of such ligands are shown to produce hexene-1 at very high productivities and selectivities.

For convenience, the ligands above might generally be referred to as P-N-P ligands and the preferred ligands (where each X is a phenyl) might be referred to as tetraphenyl P-N-P ligands. It has since been discovered that seemingly small changes to the substitution pattern on the phenyl groups of these ligands can produce very large differences in the productivity and selectivity of Cr catalysts prepared with the P-N-P ligands.

Selective oligomerizations using similar ligands are disclosed in US patents 7,297,832 (Dixon to Sasol); 7,273,959 (Drent et al. to Shell); 7,378,537 (Small et al. to Chevron Phillips); and WO2008/11915 (Gao et al. to NOVA Chemicals). Other polydentate, heteroatom ligands for selective oligomerizations are disclosed in US 7,414,006 (M°Conville et al. to Exxon).

The above described polydendate ligands generally provide high activity and selectivity, particularly in comparison to the original catalysts of Briggs. However, polymer formation may still be observed with these ligands, particularly when using a liquid filled continuously stirred tank reactor (CSTR). We have further observed particular problems with polymer formation during continuous reactor operations (as opposed to batch operation).

The use of hydrogen to mitigate polymer production has previously been disclosed. In particular, Wass (US 6,800,702) illustrates the use of hydrogen at example 14 and Dixon (US 7,297,832) does likewise at example 15. However, we have still observed some production of polymer when operating a continuously stirred liquid full reactor with added hydrogen and the process of this invention mitigates this problem.

### DISCLOSURE OF INVENTION

The present invention provides:
A continuous process for the oligomerization of ethylene in a liquid full, continuously stirred tank reactor, comprising the steps of:
   a) feeding ethylene, hydrogen and an oligomerization catalyst system to said reactor;
   b) oligomerizing said ethylene in the presence of said catalyst system and said hydrogen, characterized in that
   c) said ethylene and said hydrogen are contacted prior to being added to said reactor and are jointly added to said reactor via a common feed tube.

The use of hydrogen is essential to this invention. The use of from 200 to 20,000 parts per million by weight (based on the weight of ethylene) is preferred, especially from 500 to 10,000 ppm.

### BEST MODE FOR CARRYING OUT THE INVENTION

### Part A: Reactor and Reactor Conditions

The process of this invention uses a conventional stirred tank reactor ("CSTR"). Furthermore, the reactor is operated in a "continuous mode". The term "continuous mode" as used herein means that feed streams (such as monomers, catalyst and solvent or diluents) are added to the reactor at the same time as product streams (containing oligomerization product) are being withdrawn.

Most preferably, the reactor is operated in a "liquid full" mode (which, as used herein, means that the reactor is at least 90% full of liquid).

For clarity, this invention does not include the following types of reactors (all of which have been used for ethylene oligomerizations or have been proposed for such use in the patent literature):
1) Batch reactors;
2) Gas phase reactors;
3) Catalytic distillation reactors; and
4) Plug flow reactors (i.e. tubular or serpentine reactors that are not stirred).

More specifically, the process of this invention mitigates a problem that occurs with CSTRs operated in continuous mode, namely the formation of polymer (especially polymer that is formed adjacent to the ethylene feed stream port).

Oligomerization reactors may generally be operated adiabaticly, or alternatively, with added heating or cooling.

The present invention is preferably operated with a cooling system to remove some of the heat of reaction. The continuous reactor used in the Examples is equipped with an external cooling jacket. Larger reactors would preferably be equipped with cooling coils that are in contact with the liquid contained in the reactor. These cooling coils may be located inside the reactor, outside the reactor or both.

In a preferred embodiment, the reactor is operated in an "isothermal" manner - i.e. the reactor temperature is controlled around an aiming point temperature. It is especially preferred that the isothermal operation is controlled to within ± 5° C of the aiming point. It is preferred to operate the oligomerization reaction at a temperature of from 10° C to 100° C (especially 20° C to 80° C) and a pressure of up to 50 MPa (especially from 2 to 10 MPa).

In a highly preferred embodiment, hydrogen is added to the reactor prior to the introduction of ethylene and catalyst - in other words, the reactor is "pre-charged" with hydrogen prior to the start of the oligomerization reaction. Hydrogen is also added during the continuous operation of the reaction. The hydrogen is added by way of the same tube that provides the ethylene. It is highly preferred that a continuous flow of ethylene and hydrogen be provided but an intermittent or pulse flow of both may also be employed. Preferred amounts of hydrogen are comparatively small - from 200 to 20,000 parts per million by weight (based on the weight of ethylene), especially from 500 to 5000 ppm.

### Part B: Catalyst System

A catalyst system which produces oligomers of ethylene and by-product polyethylene is used in the present invention.

The catalyst system used in the process of the present invention contains three components, namely:
(i) a source of chromium:
(ii) an oligomerization active ligand comprising anamine; and
(iii) an activator.

Preferred forms of each of these components are discussed below.

### Chromium Source ("Component (i)")

Any source of chromium which allows the oligomerization process of the present invention to proceed may be used. Preferred chromium sources include chromium trichloride; chromium (III) 2-ethylhexanoate; chromium (III) acetylacetonate and chromium carboxyl complexes such as chromium hexacarboxyl. The oligomerization process of this invention is preferably conducted in a liquid that is a solvent for the chromium source.

### Ligand Used in the Oligomerization Process ("Component (ii)")

Anoligomerization ligand which produces oligomers of ethylene and by-product polyethylene is used in this invention. Ligands used in the oligomerization process of this invention are defined by the formula: 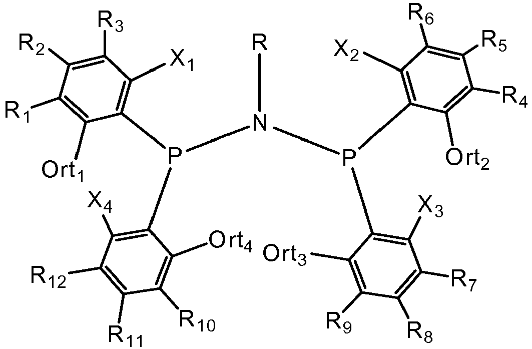 wherein each of X₁ to X₄ is independently selected from the group consisting of F, Br, H, Cl, a "polar group" and a hydrocarbyl group having from 1 to 30 carbon atoms; and wherein each of R; ort¹ to ort⁴ and R₁ to R₁₂ is independently selected from the group consisting of H and "non-interfering substituents" (i.e. substituents that do not interfere with the oligomerization reaction).

Each of X₁ to X₄ is most preferably fluorine.

R is preferably a hydrocarbyl group having from 1 to 30 carbon atoms. The hydrocarbyl groups may contain heteroatom substituents (having a heteroatom selected from O, N, P and S). Simple alkyl groups having from 1 to 12 carbon atoms are preferred. Isopropyl is particularly preferred.

The term "polar group" as used herein refers to the IUPAC definition, namely that the polar group has a permanent electron dipole moment. Examples include methoxy, ethoxy, isopropoxy, C₄₋₂₀ aloxy, C₆ to C₂₀ aryloxy, amino and phosphine.

Each of ort¹ to ort⁴ is preferably selected from the group consisting of H, Br, Cl, F and small alkyl groups (having from 1 to 3 carbon atoms). H is most preferred.

Each of R₁ to R₁₂ is independently selected from the group consisting of H, hydrocarbyl substituents which contain from 1 to 30 carbon atoms (which hydrocarbyl substituents may contain heteroatoms selected from the group consisting of O, N, P and S) and heteroatom substituents (i.e. substituents which are bonded to the phenyl ring via an O, N, P or S atom) which contain from 2 to 30 atoms. It is preferred that R₁ to R₁₂ are not polar substituents. It is especially preferred that each of R₁ to R₁₂ is selected from H and C₁ to C₆ alkyls or C₆ to C₁₂ aryls.

### Activator "Component (iii)")

The activator (component (iii)) may be any compound that generates an active catalyst for ethylene oligomerization with components (i) and (ii). Mixtures of activators may also be used. Suitable compounds include organoaluminum compounds, organoboron compounds and inorganic acids and salts, such as tetrafluoroboric acid etherate, silver tetrafluoroborate, sodium hexafluoroantimonate Suitable organoaluminium compounds include compounds of the formula AIR₃, where each R is independently C₁-C₁₂ alkyl, oxygen or halide, and compounds such as LiAlH₄ and the like. Examples include trimethylaluminium (TMA), triethylaluminium (TEA), triisobutylaluminium (TIBA), tri-n-octylaluminium, methylaluminium dichloride, ethylaluminium dichloride, dimethylaluminium chloride, diethylaluminium chloride, ethylaluminiumsesquichloride, methylaluminiumsesquichloride, and alumoxanes. Alumoxanes are well known in the art as typically oligomeric compounds which can be prepared by the controlled addition of water to an alkylaluminium compound, for example trimethylaluminium. Such compounds can be linear, cyclic, cages or mixtures thereof. Commercially available alumoxanes are generally believed to be mixtures of linear and cyclic compounds. The cyclic alumoxanes can be represented by the formula [R⁶AlO]*ₛ* and the linear alumoxanes by the formula R⁷(R⁸AlO)*ₛ* wherein s is a number from about 2 to 50, and wherein R⁶, R⁷, and R⁸ represent hydrocarbyl groups, preferably C₁ to C₆ alkyl groups, for example methyl, ethyl or butyl groups. Alkylalumoxanes especially methylalumoxane (MAO) are preferred.

It will be recognized by those skilled in the art that commercially available alkylalumoxanes may contain a proportion of trialkylaluminium. For instance, commercial MAO usually contains approximately 10 wt % trimethylaluminium (TMA), and commercial "modified MAO" (or "MMAO") contains both TMA and TIBA. Quantities of alkylalumoxane are generally quoted herein on a molar basis of aluminium (and include such "free" trialkylaluminium).

Examples of suitable organoboron compounds are boroxines, NaBH₄, trimethylboron, triethylboron, dimethylphenylammoniumtetra(phenyl)borate, trityltetra(phenyl)borate, triphenylboron, dimethylphenylammonium tetra(pentafluorophenyl)borate, sodium tetrakis[(bis-3,5-trifluoromethyl)phenyl]borate, trityltetra(pentafluorophenyl)borate and tris(pentafluorophenyl) boron.

Activator compound (iii) may also be or contain a compound that acts as a reducing or oxidizing agent, such as sodium or zinc metal and the like, or oxygen and the like.

In the preparation of the catalyst systems used in the present invention, the quantity of activating compound to be employed is easily determined by simple testing, for example, by the preparation of small test samples which can be used to oligomerize small quantities of ethylene and thus to determine the activity of the produced catalyst. It is generally found that the quantity employed is sufficient to provide 0.5 to 1000 moles of aluminium (or boron) per mole of chromium. MAO is the presently preferred activator. Molar Al/Cr ratios of from 1/1 to 500/1 are preferred.

### Part C: Process Conditions

The chromium (component (i)) and ligand (component (ii)) may be present in any molar ratio which produces oligomer, preferably between 100:1 and 1:100, and most preferably from 10:1 to 1:10, particularly 3:1 to 1:3. Generally the amounts of (i) and (ii) are approximately equal, i.e. a ratio of between 1.5:1 and 1:1.5.

Components (i)-(iii) of the catalyst system utilized in the present invention may be added together simultaneously or sequentially, in any order, and in the presence or absence of ethylene in any suitable solvent, so as to give an active catalyst. For example, components (i), (ii) and (iii) and ethylene may be contacted together simultaneously, or components (i), (ii) and (iii) may be added together simultaneously or sequentially in any order and then contacted with ethylene, or components (i) and (ii) may be added together to form an isolable metal-ligand complex and then added to component (iii) and contacted with ethylene, or components (i), (ii) and (iii) may be added together to form an isolable metal-ligand complex and then contacted with ethylene. Suitable solvents for contacting the components of the catalyst or catalyst system include, but are not limited to, hydrocarbon solvents such as heptane, toluene, 1-hexene and the like, and polar solvents such as diethyl ether, tetrahydrofuran, acetonitrile, dichloromethane, chloroform, chlorobenzene

The catalyst components (i), (ii) and (iii) utilized in the present invention can be unsupported or supported on a support material, for example, silica, alumina, MgCl₂ or zirconia, or on a polymer, for example polyethylene, polypropylene, polystyrene, or poly(aminostyrene). If desired the catalysts can be formed in situ in the presence of the support material or the support material can be pre-impregnated or premixed, simultaneously or sequentially, with one or more of the catalyst components. The quantity of support material employed can vary widely, for example from 100,000 to 1 grams per gram of metal present in the transition metal compound. In some cases, the support may material can also act as or as a component of the activator compound (iii). Examples include supports containing alumoxane moieties.

The oligomerization can be conducted under solution phase, slurry phase or bulk phase conditions. Suitable temperatures range from 10° C to +300° C preferably from 10° C to 100° C, especially from 20 to 80° C. Suitable pressures are from atmospheric to 50 MPa (gauge) preferably from 2 to 10 MPa.

Irrespective of the process conditions employed, the oligomerization is typically carried out under conditions that substantially exclude oxygen, water, and other materials that act as catalyst poisons. Also, oligomerization can be carried out in the presence of additives to control selectivity, enhance activity and reduce the amount of polymer formed in oligomerization processes. Potentially suitable additives include a halide source. Also advantageous may be a process which includes more than one reactor, a catalyst kill system between reactors or after the final reactor, or an integrated reactor/separator/purifier. While all catalyst components, reactants, inerts, and products could be employed in the present invention on a once-through basis, it is often economically advantageous to recycle one or more of these materials; in the case of the catalyst system, this might require reconstituting one or more of the catalysts components to achieve the active catalyst system. It is within the scope of this invention that an oligomerization product might also serve as a solvent or diluent. Mixtures of inert diluents or solvents also could be employed. The preferred diluents or solvents are aliphatic and aromatic hydrocarbons and halogenated hydrocarbons such as, for example, isobutane, pentane, toluene, xylene, ethylbenzene, cumene, mesitylene, heptane, cyclohexane, methylcyclohexane, 1-hexene, 1-octene, chlorobenzene, dichlorobenzene, and the like, and mixtures such as Isopar™.

Techniques for varying the distribution of products from the oligomerization reactions include controlling process conditions (e.g. concentration of components (i)-(iii), reaction temperature, pressure, residence time) and properly selecting the design of the process and are well known to those skilled in the art.

The ethylene feedstock for the oligomerization may be substantially pure or may contain other olefinic impurities and/or ethane. One embodiment of the process of the invention comprises the oligomerization of ethylene-containing waste streams from other chemical processes or a crude ethylene/ethane mixture from a cracker.

In a preferred embodiment of the present invention, the oligomerization product produced from this invention is added to a product stream from another alpha olefins manufacturing process for separation into different alpha olefins. As previously discussed, "conventional alpha olefin plants" (wherein the term includes i) those processes which produce alpha olefins by a chain growth process using an aluminum alkyl catalyst, ii) the aforementioned "SHOP" process and iii) the production of olefins from synthesis gas using the so called Lurgi process) have a series of distillation columns to separate the "crude alpha product" (i.e. a mixture of alpha olefins) into alpha olefins (such as butene-1, hexene-1 and octene-1). The mixed hexene-octene product which is produced in accordance with the present invention is highly suitable for addition/mixing with a crude alpha olefin product from an existing alpha olefin plant (or a "cut" or fraction of the product from such a plant) because the mixed hexene-octene product produced in accordance with the present invention can have very low levels of internal olefins. Thus, the hexene-octene product of the present invention can be readily separated in the existing distillation columns of alpha olefin plants (without causing the large burden on the operation of these distillation columns which would otherwise exist if the present hexene-octene product stream contained large quantities of internal olefins). As used herein, the term "liquid product" is meant to refer to the oligomers produced by the process of the present invention which have from 4 to (about) 20 carbon atoms.

The liquid product from the oligomerization process of the present invention preferably consists of from 20 to 80 weight% octenes (especially from 35 to 75 weight%) octenes and from 15 to 50 weight% (especially from 20 to 40 weight%) hexenes (where all of the weight% are calculated on the basis of the liquid product by 100%.

The preferred oligomerization process of this invention is also characterized by producing very low levels of internal olefins (i.e. low levels of hexene-2, hexene-3, octene-2, octene-3 etc.), with preferred levels of less than 10 weight% (especially less than 5 weight%) of the hexenes and octenes being internal olefins.

One embodiment of the present invention encompasses the use of components (i) (ii) and (iii) in conjunction with one or more types of olefin polymerization catalyst system (iv) to oligomerize ethylene and subsequently incorporate a portion of the oligomerization product(s) into a higher polymer.

Component (iv) may be one or more suitable polymerization catalyst system(s), examples of which include, but are not limited to, conventional Ziegler-Natta catalysts, metallocene catalysts, monocyclopentadienyl or "constrained geometry" catalysts, phosphinimine catalysts, heat activated supported chromium oxide catalysts (e.g. "Phillips"-type catalysts), late transition metal polymerization catalysts (eg. diimine, diphosphine and salicylaldimine nickel/palladium catalysts, iron and cobalt pyridyldiimine catalysts and the like) and other so-called "single site catalysts" (SSC's).

Ziegler-Natta catalysts, in general, consist of two main components. One component is an alkyl or hydride of a Group I to III metal, most commonly Al(Et)₃ or Al(iBu)₃ or Al(Et)₂Cl but also encompassing Grignard reagents, n-butyllithium, or dialkylzinc compounds. The second component is a salt of a Group IV to VIII transition metal, most commonly halides of titanium or vanadium such as TiCl₄, TiCl₃, VCl₄, or VOCl₃. The catalyst components when mixed, usually in a hydrocarbon solvent, may form a homogeneous or heterogeneous product. Such catalysts may be impregnated on a support, if desired, by means known to those skilled in the art and so used in any of the major processes known for co-ordination catalysis of polyolefins such as solution, slurry, and gas-phase. In addition to the two major components described above, amounts of other compounds (typically electron donors) maybe added to further modify the polymerization behaviour or activity of the catalyst.

Metallocene catalysts, in general, consist of transition metal complexes, most commonly based on Group IV metals, ligated with cyclopentadienyl(Cp)-type groups. A wide range of structures of this type of catalysts is known, including those with substituted, linked and/or heteroatom-containing Cp groups, Cp groups fused to other ring systems and the like. Additional activators, such as boranes or alumoxane, are often used and the catalysts may be supported, if desired.

Monocyclopentadienyl or "constrained geometry" catalysts, in general, consist of a transition metal complexes, most commonly based on Group IV metals, ligated with one cyclopentadienyl(Cp)-type group, often linked to additional donor group. A wide range of structures of this type of catalyst is known, including those with substituted, linked and/or heteroatom-containing Cp groups, Cp groups fused to other ring systems and a range of linked and non-linked additional donor groups such as amides, amines and alkoxides. Additional activators, such as boranes or alumoxane, are often used and the catalysts may be supported, if desired.

A typical heat activated chromium oxide (Phillips) type catalyst employs a combination of a support material to which has first been added a chromium-containing material wherein at least part of the chromium is in the hexavalent state by heating in the presence of molecular oxygen. The support is generally composed of about 80 to 100 wt. % silica, the remainder, if any, being selected from the group consisting of refractory metal oxides, such as aluminium, boria, magnesia, thoria, zirconia, titania and mixtures of two or more of these refractory metal oxides. Supports can also comprise alumina, aluminium phosphate, boron phosphate and mixtures thereof with each other or with silica. The chromium compound is typically added to the support as a chromium (III) compound such as the acetate or acetylacetonate in order to avoid the toxicity of chromium (VI). The raw catalyst is then calcined in air at a temperature between 250 and 1000° C. for a period of from a few seconds to several hours. This converts at least part of the chromium to the hexavalent state. Reduction of the Cr (VI) to its active form normally occurs in the polymerization reaction, but can be done at the end of the calcination cycle with CO at about 350° C. Additional compounds, such as fluorine, aluminium and/or titanium may be added to the raw Phillips catalyst to modify it.

Late transition metal and single site catalysts cover a wide range of catalyst structures based on metals across the transition series.

Component (iv) may also comprise one or more polymerization catalysts or catalyst systems together with one or more additional oligomerization catalysts or catalyst systems. Suitable oligomerization catalysts include, but are not limited to, those that dimerise (for example, nickel phosphine dimerisation catalysts) or trimerise olefins or otherwise oligomerize olefins to, for example, a broader distribution of 1-olefins (for example, iron and cobalt pyridyldiimine oligomerization catalysts).

Component (iv) may independently be supported or unsupported. Where components (i) and (ii) and optionally (iii) are supported, (iv) may be co-supported sequentially in any order or simultaneously on the same support or may be on a separate support. For some combinations, the components (i) (iii) may be part or all of component (iv). For example, if component (iv) is a heat activated chromium oxide catalyst then this may be (i), a chromium source and if component (iv) contains an alumoxane activator then this may also be the optional activator (iii).

The components (i), (ii), (iii) and (iv) may be in essentially any molar ratio that produces a polymer product. The precise ratio required depends on the relative reactivity of the components and also on the desired properties of the product or catalyst systems.

An "in series" process could be conducted by first conducting the oligomerization reaction, then passing the oligomerization product to a polymerization reaction. In the case of an "in series" process various purification, analysis and control steps for the oligomeric product could potentially be incorporated between the trimerization and subsequent reaction stages. Recycling between reactors configured in series is also possible. An example of such a process would be the oligomerization of ethylene in a single reactor with a catalyst comprising components (i)-(iii) followed by copolymerization of the oligomerization product with ethylene in a separate, linked reactor to give branched polyethylene. Another example would be the oligomerization of an ethylene-containing waste stream from a polyethylene process, followed by introduction of the oligomerization product back into the polyethylene process as a co-monomer for the production of branched polyethylene.

An example of an "in situ" process is the production of branched polyethylene catalyzed by components (i)-(iv), added in any order such that the active catalytic species derived from components (i)-(iii) are at some point present in a reactor with component (iv).

Both the "in series and "in situ" approaches can be adaptions of current polymerization technology for the process stages including component (iv). All major olefin existing polymerization processes, including multiple reactor processes, are considered adaptable to this approach. One adaption is the incorporation of an oligomerization catalyst bed into a recycle loop of a gas phase polymerization process, this could be as a side or recycle stream within the main fluidization recycle loop and or within the degassing recovery and recycle system.

Polymerization conditions when component (iv) is present can be, for example, solution phase, slurry phase, gas phase or bulk phase, with temperatures ranging from -100° C to +300° C and at pressures of atmospheric and above, particularly from 1.5 to 50 atmospheres. Reaction conditions, will typically have a significant impact upon the properties (e.g. density, melt index, yield) of the polymer being made and it is likely that the polymer requirements will dictate many of the reaction variables. Reaction temperature, particularly in processes where it is important to operate below the sintering temperature of the polymer, will typically, and preferably, be primarily selected to optimize the polymerization reaction conditions. Also, polymerization or copolymerization can be carried out in the presence of additives to control polymer or copolymer molecular weights. The use of hydrogen gas as a means of controlling the average molecular weight of the polymer or copolymer applies generally to the polymerization process of the present invention.

Slurry phase polymerization conditions or gas phase polymerization conditions are particularly useful for the production of high or low density grades of polyethylene, and polypropylene. In these processes the polymerization conditions can be batch, continuous or semi-continuous. Furthermore, one or more reactors may be used, e.g. from two to five reactors in series. Different reaction conditions, such as different temperatures or hydrogen concentrations may be employed in the different reactors.

Once the polymer product is discharged from the reactor, any associated and absorbed hydrocarbons are substantially removed, or degassed, from the polymer by, for example, pressure let-down or gas purging using fresh or recycled steam, nitrogen or light hydrocarbons (such as ethylene). Recovered gaseous or liquid hydrocarbons may be recycled to a purification system or the polymerization zone.

In the slurry phase polymerization process the polymerization diluent is compatible with the polymer(s) and catalysts, and may be an alkane such as hexane, heptane, isobutane, or a mixture of hydrocarbons or paraffins. The polymerization zone can be, for example, an autoclave or similar reaction vessel, or a continuous liquid full loop reactor, e.g. of the type well-known in the manufacture of polyethylene by the Phillips Process. When the polymerization process of the present invention is carried out under slurry conditions the polymerization is preferably carried out at a temperature above 0° C, most preferably above 15° C. Under slurry conditions the polymerization temperature is preferably maintained below the temperature at which the polymer commences to soften or sinter in the presence of the polymerization diluent. If the temperature is allowed to go above the latter temperature, fouling of the reactor can occur. Adjustment of the polymerization within these defined temperature ranges can provide a useful means of controlling the average molecular weight of the produced polymer. A further useful means of controlling the molecular weight is to conduct the polymerization in the presence of hydrogen gas which acts as chain transfer agent. Generally, the higher the concentration of hydrogen employed, the lower the average molecular weight of the produced polymer.

In bulk polymerization processes, liquid monomer such as propylene is used as the polymerization medium.

Methods for operating gas phase polymerization processes are well known in the art. Such methods generally involve agitating (e.g. by stirring, vibrating or fluidizing) a bed of catalyst, or a bed of the target polymer (i.e. polymer having the same or similar physical properties to that which it is desired to make in the polymerization process) containing a catalyst, and feeding thereto a stream of monomer (under conditions such that at least part of the monomer polymerizes in contact with the catalyst in the bed. The bed is generally cooled by the addition of cool gas (e.g. recycled gaseous monomer) and/or volatile liquid (e.g. a volatile inert hydrocarbon, or gaseous monomer which has been condensed to form a liquid). The polymer produced in, and isolated from, gas phase processes forms directly a solid in the polymerization zone and is free from, or substantially free from liquid. As is well known to those skilled in the art, if any liquid is allowed to enter the polymerization zone of a gas phase polymerization process the quantity of liquid in the polymerization zone is small in relation to the quantity of polymer present. This is in contrast to "solution phase" processes wherein the polymer is formed dissolved in a solvent, and "slurry phase" processes wherein the polymer forms as a suspension in a liquid diluent.

The gas phase process can be operated under batch, semi-batch, or so-called "continuous" conditions. It is preferred to operate under conditions such that monomer is continuously recycled to an agitated polymerization zone containing polymerization catalyst, make-up monomer being provided to replace polymerized monomer, and continuously or intermittently withdrawing produced polymer from the polymerization zone at a rate comparable to the rate of formation of the polymer, fresh catalyst being added to the polymerization zone to replace the catalyst withdrawn from the polymerization zone with the produced polymer.

Methods for operating gas phase fluidized bed processes for making polyethylene, ethylene copolymers and polypropylene are well known in the art. The process can be operated, for example, in a vertical cylindrical reactor equipped with a perforated distribution plate to support the bed and to distribute the incoming fluidizing gas stream through the bed. The fluidizing gas circulating through the bed serves to remove the heat of polymerization from the bed and to supply monomer for polymerization in the bed. Thus the fluidizing gas generally comprises the monomer(s) normally together with some inert gas (e.g. nitrogen or inert hydrocarbons such as methane, ethane, propane, butane, pentane or hexane) and optionally with hydrogen as molecular weight modifier. The hot fluidizing gas emerging from the top of the bed is led optionally through a velocity reduction zone (this can be a cylindrical portion of the reactor having a wider diameter) and, if desired, a cyclone and or filters to disentrain fine solid particles from the gas stream. The hot gas is then led to a heat exchanger to remove at least part of the heat of polymerization. Catalysts are preferably fed continuously or at regular intervals to the bed. At start up of the process, the bed comprises fluidizable polymer which is preferably similar to the target polymer. Polymer is produced continuously within the bed by the polymerization of the monomer(s). Preferably means are provided to discharge polymer from the bed continuously or at regular intervals to maintain the fluidized bed at the desired height. The process is generally operated at relatively low pressure, for example, at 10 to 50 atmospheres, and at temperatures for example, between 50 and 135° C. The temperature of the bed is maintained below the sintering temperature of the fluidized polymer to avoid problems of agglomeration.

In the gas phase fluidized bed process for polymerization of olefins the heat evolved by the exothermic polymerization reaction is normally removed from the polymerization zone (i.e. the fluidized bed) by means of the fluidizing gas stream as described above. The hot reactor gas emerging from the top of the bed is led through one or more heat exchangers wherein the gas is cooled. The cooled reactor gas, together with any make-up gas, is then recycled to the base of the bed. In the gas phase fluidized bed polymerization process of the present invention it is desirable to provide additional cooling of the bed (and thereby improve the space time yield of the process) by feeding a volatile liquid to the bed under conditions such that the liquid evaporates in the bed thereby absorbing additional heat of polymerization from the bed by the "latent heat of evaporation" effect. When the hot recycle gas from the bed enters the heat exchanger, the volatile liquid can condense out. In one embodiment of the present invention the volatile liquid is separated from the recycle gas and reintroduced separately into the bed. Thus, for example, the volatile liquid can be separated and sprayed into the bed. In another embodiment of the present invention the volatile liquid is recycled to the bed with the recycle gas. Thus the volatile liquid can be condensed from the fluidizing gas stream emerging from the reactor and can be recycled to the bed with recycle gas, or can be separated from the recycle gas and then returned to the bed.

A number of process options can be envisaged when using the catalysts of the present invention in an integrated process to prepare higher polymers i.e. when component (iv) is present. These options include "in series" processes in which the oligomerization and subsequent polymerization are carried in separate but linked reactors and "in situ" processes in which a both reaction steps are carried out in the same reactor.

In the case of a gas phase "in situ" polymerization process, component (iv) can, for example, be introduced into the polymerization reaction zone in liquid form, for example, as a solution in a substantially inert liquid diluent. Components (i)-(iv) may be independently added to any part of the polymerization reactor simultaneously or sequentially together or separately. Under these circumstances it is preferred the liquid containing the component(s) is sprayed as fine droplets into the polymerization zone. The droplet diameter is preferably within the range 1 to 1000 microns.

Although not usually required, upon completion of polymerization or copolymerization, or when it is desired to terminate polymerization or copolymerization or at least temporarily deactivate the catalyst or catalyst component of this invention, the catalyst can be contacted with water, alcohols, acetone, or other suitable catalyst deactivators a manner known to persons of skill in the art.

A range of polyethylene polymers are considered accessible including high density polyethylene, medium density polyethylene, low density polyethylene, ultra low density polyethylene and elastomeric materials. Particularly important are the polymers having a density in the range of 0.91 to 0.93, grams per cubic centimeter (g/cc) generally referred to in the art as linear low density polyethylene. Such polymers and copolymers are used extensively in the manufacture of flexible blown or cast film.

Depending upon the use of the polymer product, minor amounts of additives are typically incorporated into the polymer formulation such as acid scavengers, antioxidants, stabilizers, and the like. Generally, these additives are incorporated at levels of about 25 to 2000 parts per million by weight (ppm), typically from about 50 to about 1000 ppm, and more typically 400 to 1000 ppm, based on the polymer. In use, polymers or copolymers made according to the invention in the form of a powder are conventionally compounded into pellets. Examples of uses for polymer compositions made according to the invention include use to form fibres, extruded films, tapes, spunbonded webs, molded or thermoformed products, and the like. The polymers may be blown or cast into films, or may be used for making a variety of molded or extruded articles such as pipes, and containers such as bottles or drums. Specific additive packages for each application may be selected as known in the art. Examples of supplemental additives include slip agents, anti-blocks, anti-stats, mould release agents, primary and secondary anti-oxidants, clarifiers, nucleants, uv stabilizers, and the like. Classes of additives are well known in the art and include phosphite antioxidants, hydroxylamine (such as N,N-dialkyl hydroxylamine) and amine oxide (such as dialkyl methyl amine oxide) antioxidants, hindered amine light (uv) stabilizers, phenolic stabilizers, benzofuranone stabilizers, and the like.

Fillers such as silica, glass fibers, talc, and the like, nucleating agents, and colourants also may be added to the polymer compositions as known by the art.

The present invention is illustrated in more detail by the following non-limiting examples.

### EXAMPLES

The following abbreviations are used in the examples:
A = Angstrom units
NMR = nuclear magnetic resonance
Et = ethyl
Bu = butyl
iPr = isopropyl
c^{*} = comparative
rpm = revolutions per minute
GC = gas chromatography
Rₓ = reaction
Wt = weight
C₄'s = butenes
C₆'s = hexenes
C₈'s = octenes
PE = polyethylene

### Part I: Preferred Ligand Synthesis

### General

All reactions involving air and or moisture sensitive compounds were conducted under nitrogen using standard Schlenk or cannula techniques, or in a glovebox. Reaction solvents were purified prior to use (e.g. by distillation) and stored over activated 4 A sieves. Diethylamine, triethylamine and isopropylamine were purchased from Aldrich and dried over 4 A molecular sieves prior to use. 1-Bromo-2-fluorobenzene, phosphorus trichloride (PCl₃), hydrogen chloride gas and n-butyllithium were purchased from Aldrich and used as is. The methylalumoxane (MAO), 10 wt% Al in toluene, was purchased from Akzo and used as is. Deuterated solvents were purchased (toluene-d₈, THF-d₈) and were stored over 4 A sieves. NMR spectra were recorded on a Bruker 300 MHz spectrometer (300.1 MHz for ¹H, 121.5 MHz for ³¹P, 282.4 for ⁹F).

### Preparation of Et₂NPCl₂

Et₂NH (50.00 mmol, 5.17 mL) was added dropwise to a solution of PCl₃ (25.00 mmol, 2.18 mL) in diethyl ether (will use "ether" from here) (200 mL) at -78° C. After the addition, the cold bath was removed and the slurry was allowed to warm to room temperature over 2 hours. The slurry was filtered and the filtrate was pumped to dryness. The residue was distilled (500 microns, 55° C) to give the product in quantitative yield.
¹H NMR (δ, toluene-d₈): 2.66 (doublet of a quartets, 4H, J_{PH} = 13 Hz, J_{HH} = 7 Hz), 0.75 (triplet, 6H, J = 7 Hz).

### Preparation of (ortho-F-C₆H₄)₂P-NEt₂

To solution of n-BuLi (17.00 mL of 1.6 M n-BuLi hexane solution, 27.18 mmol) in ether (100 mL) maintained at -85° C, was added dropwise a solution of 1-bromo-2-fluorobenzene (4.76 g, 27.18 mmol) in ether (40 mL) over 2 hours. After addition, the reaction flask was stirred for 1 hour at -78° C, resulting in a white slurry. Et₂NPCl₂ (2.36 g, 13.58 mmol) in ether (20 mL) was then added very slowly while the reaction temperature was maintained at -85° C. The reaction was allowed to warm to -10° C overnight. Toluene (10 mL) was then added to the reaction flask and the volatiles were removed *in vacuo.* The residue was extracted with toluene and the solution was pumped to dryness. The crude product was distilled (300 microns, 100°C) yielding 3.78 g (95%) of product. ¹H NMR (δ, THF-d₈): 7.40-7.01 (4 equal intense multiplets, 8H), 3.11 (doublets of quartet, 4H, J_{PH} = 13 Hz, J_{HH} = 7 Hz), 0.97 (triplet, 6H, J = 7 Hz). ¹⁹F NMR (δ, THF-d₈): -163.21 (doublet of multiplets, J = 48 Hz). GC-MS. M⁺ = 293.

### Preparation of (ortho-F-C₆H₄)PCl

Anhydrous HCl_{(g)} was introduced to the head space of an ethereal solution (100 mL) of (*ortho*-F-C₆H₄)P-NEt₂ (3.73 g, 12.70 mmol) to a pressure of 3 psi. A white precipitate formed immediately. The reaction was stirred for an additional 0.5 hours at which point the slurry was pumped to dryness to remove volatiles. The residue was re-slurried in ether (100 mL) and filtered. The filtrate was pumped to dryness yielding (*ortho*-F-C₆H₄)₂PCl as a colorless oil in quantitative yield. ¹H NMR (δ, THF-d₈): 7.60 (m, 4H), 7.20 (m, 2H), 7.08 (m, 2H). ¹⁹F NMR (δ, THF-d₈): -106.94 (doublet of multiplets, J = 67 Hz).

### Preparation of (ortho-F-C₆H₄)₂PNH(i-Pr)

To a solution of (*ortho*-F-C₆H₄)PCl (1.00 g, 3.90 mmol) in ether (50 mL) and NEt₃ (3 mL) was added an ethereal solution of i-PrNH₂ (0.42 mL, 4.90 mmol) at -5 °C. Immediate precipitate was observed. The slurry was stirred for 3 hours and filtered. The filtrate was pumped to dryness to give a colorless oil of (*ortho-*F-C₆H₄)PNH(i-Pr) in quantitative yield.
¹H NMR (δ, THF-d₈): 7.42 (m, 2H), 7.30 (m, 2H), 7.11 (m, 2H), 6.96 (m, 2H), 3.30 (septet, 1H, J= 7 Hz), 2.86 (br s, 1H), 1.15 (d, 6H, J = 7 Hz). ¹⁹F NMR (δ, THF-d₈): - 109.85 (doublet of multiplets, J = 40 Hz). GC-MS, M⁺ = 279.

### Preparation of (ortho-F-C₆H₄)₂PN(i-Pr)P(ortho-F-C₆H₄)₂ ("Ligand 1")

To a solution of (*ortho*-F-C₆H₄)₂PNH(i-Pr) (3.90 mmol) [made from i-PrNH₂ and (*ortho*-F-C₆H₄)₂PCl (1.00 g, 3.90 mmol)] in ether (100 mL) maintained at -70 °C was added dropwise a solution of n-BuLi (2.43 mL of 1.6 M n-BuLi hexane solution, 3.90 mmol)). The mixture was stirred at -70° C for 1 hour and allowed to warm to -10°C in a cold bath (2 hours). The solution was re-cooled to -70° C and (*ortho*-F-C₆H₄)₂PCl (1.00 g, 3.90 mmol) was slowly added. The solution was stirred for 1 hour at -70° C and allowed to slowly warm to room temperature forming a white precipitate. The slurry was pumped to dryness and the residue was extracted with toluene and filtered. The filtrate was pumped to dryness and recrystallized from heptane at -70° C (2x) yielding 1.13 g (58%) of product. At room temperature this material was an oil which contained both the desired ligand (ortho-F-C₆H₄)₂PN(i-Pr)P(ortho-F-C₆H₄)₂ and its isomer (ortho-F-C₆H₄)₂P[=N(i-Pr]P(ortho-F-C₆H₄)₂. A toluene solution of this mixture and 50 mg of (ortho-F-C₆H₄)₂PCl was heated at 65 °C for three hours to convert the isomer to the desired ligand. ¹H NMR (THF-d8, δ): 7.35 (m, 8H), 7.10 (m, 4H), 6.96 (m, 4H), 3.94 (m, 1H), 1.24 (d, 6H, J = 7Hz). ¹⁹F NMR (THF-d₈, δ): -104.2 (br. s).

In a more preferred procedure the initial steps of the synthesis are conducted in pentane at -5° C (instead of ether) with 10% more of the (ortho-F-C₆H₄)₂PCl (otherwise as described above). This preferred procedure allows (ortho-F-C₆H₄)₂PN(i-Pr)P(ortho-F-C₆H₄)₂ to be formed in high (essentially quantitative) yield without the final step of heating in toluene.

### Catalyst Preparation

The term catalyst refers to the chromium molecule with the heteroatom ligand bonded in place. The preferred P-N-P ligand does not easily react with some Cr (III) molecules - especially when using the most preferred P-N-P ligands (which ligands contain phenyl groups bonded to the P atoms, further characterized in that at least one of the phenyl groups contains an ortho fluoro substituent).

While not wishing to be bound by theory, it is believed that the reaction between the ligand and the Cr species is facilitated by aluminum alkyl or MAO. It is also believed that the reaction is facilitated by an excess of Al over Cr. Accordingly, it is most preferred to add the Cr/ligand mixture to the MAO (and/or Al alkyl) instead of the reverse addition sequence. In this manner, the initiation of the reaction is believed to be facilitated by the very high Al/Cr ratio that exists when the first part of the Cr/ligand is added to the MAO.

In a similar vein, it is believed that the ligand/Cr ratio provides another kinetic driving force for the reaction - i.e. the reaction is believed to be facilitated by high ligand/Cr ratios. Thus, one way to drive the reaction is to use an excess of ligand. In another, (preferred) reaction, a mixture with a high ligand/Cr ratio is initially employed, followed by lower ligand/Cr ratio mixtures, followed by Cr (in the absence of ligand).

### Part II: Ethylene Oligomerization

### Batch Operation (comparative)

A stirred reactor having a volume of about 600 cc was used in more than 20 (comparative) batch experiments. Chromium ("Cr", as chromium (III) acetylacetonate) plus (ortho-F-C₆H₄)₂PN(i-Pr)P(ortho-F-C₆H₄)₂ ("ligand 1", as described above) and methylaluminoxane ("MAO", purchased from Albemarle) were added to the reactor under a wide variety of conditions. In general, a Cr/ligand ratio of about 1/1 and Al/Cr ratio of from 100/1 to 500/1 were tested. Cyclohexane was used as solvent.

Ethylene was added on demand to maintain pressure but the reactor was operated in "batch" mode in the sense that product was not withdrawn and catalyst was not added during the reaction. Batch oligomerization experiments were typically operated for about 12-18 minutes.

The reaction produced hexene and octene in high yield and high selectivity over a range of conditions. Combined octene/hexene yields were typically from 400-500,000 grams of oligomer per gram of chromium per hour and represented more than 85% of the converted ethylene (i.e. less than 15 weight% of the ethylene was converted into butene plus C₁₀⁺ products). Octene/hexene ratios were generally in excess of 2/1 but less than 3/1 and the purity of both streams was typically in excess of 95% alpha olefins (i.e. only small amounts of internal olefins were produced). Polymer by-product was noted but not unduly troublesome.

### Continuous Operation

A continuous stirred tank reactor having a volume of 1000cc was used for these experiments. A range of operating conditions were tested.

Reactor temperatures between about 40° C and 80° C and pressures of about 4 to 8 MPa were tested.

The reactor was fitted with external jacket cooling. A feed preparation unit was installed to allow ethylene to be dissolved in solvent prior to being added to the reactor. The feed preparation was also equipped with a cooling jacket (to remove heat of absorbtion).

MAO was purchased as a solution of methylaluminoxine (10 weight % Al in toluene) from Albemarle.

The reactor was operated in a continuous manner - i.e. product was removed from the reactor during the reaction and make-up feed was added. Typical flow rates and reactor concentrations were as follows:
Chromium (as Cr(acac)₃): 0.025 mmol/litre
Ligand/Cr mole ratio = 1/1
Al/Cr mole ratio = 300/1 (Albemarle MAO)
Ethylene feed rate = 3 g/minute
MAO solution + cyclohexane - 33 ml/minute

The ligand fraction produced in these experiments was similar to that produced in the batch experiments - i.e. both of the octene and hexene streams were typically greater than 95% alpha olefins and octene/hexene ratio was typically at least 2/1.

Severe polymer formation was encountered during continuous operation.

A hydrogen feed line was installed for the next group of experiments. The addition of hydrogen did mitigate polymer formation and it became possible to operate the reactor over extended periods of time. It is important to note that the hydrogen was not observed to hydrogenate the feed or the products in any meaningful manner (i.e. no ethane, hexane or octane were detected).

However, after about 2-3 hours of operation, polymer build-up within the reactor typically caused instabilities in reactor control and eventual reactor shut-down. Polymer formation was especially prevalent at the exit of the ethylene feed tube. Additional experimentation was completed in which hydrogen was added to the reactor prior to the addition of ethylene and catalyst. Hydrogen was also added during the continuous operation. This mitigated polymer formation but, again, polymer formation in the ethylene feed tube was observed.

### Inventive Experiments

The feed preparation unit and reactor were then reconfigured so that the hydrogen feed line to the reactor was removed and a hydrogen feed line to the feed preparation unit was installed. In this manner, hydrogen was contacted with the ethylene and solvent prior to being introduced to the reactor and the ethylene, hydrogen and solvent were added via a common feed line.

The reconfigured unit was successfully tested for several three hour tests according to the conditions shown in Table 1. The reactor was opened at the end of the test. Many of the reactor internals were lightly coated with a fine dust like coating of polymer. A minor amount of polymer formation was observed at the very exit/outlet of the monomer/solvent feed tube but the interior of the tube was clean and the exit/outlet was not blocked (in comparison to the previous example, where a blockage of the feed tube caused reactor instability).

As shown in Table 1, no polymer was collected from the reactor for experiments 1-2. A small amount of polymer was collected in experiment 3.

Subsequent experiments were conducted using octene-1 as the reactor solvent. Again, the co-addition of ethylene and hydrogen through a common feed line prevented polymer buildup at the exit of the ethylene feed line and allowed stable operation of the reactor. Ethylene flow rates were increased to 7 g/min (run 4) and 8.5 g/min (run 5) and the H₂ flow rate was increased to 90 cubic centimeters per minute. Total reactor pressure was 8 MPa. The [Cr] concentration was 0.025 mmol/litre; Cr:ligand mole ratio was 1/1 and the Al/Cr ratio was 300/1 for both of runs 4 and 5. Three hour runs were successfully completed, with measured hexene and octene production rates as follows:
Run 4: hexene-1 (0.49 g/min); octene-1 (2.07 g/minute);
Run 5: hexene-1 (0.63 g/min); octene-1 (1.59 g/minute)
[Note: the octene-1 used as the reaction solvent was spiked with a known amount of cyclohexene to allow product formation to be determined by GC].

**TABLE 1**

| **Run #** | **Run time /h** | **C2 (g/min)** | **H2 (cc/min)** | **H2/C2 (g/g)** | **polymer (g/wet)** | **1-C8, wt%** | **1-C6, wt%** | **C8/C6 ratio** |
|---|---|---|---|---|---|---|---|---|
| 1 | 3 | 3 | 50 | 0.001487 | nothing collected | 3.19 | 2.45 | 1.3 |
| 2 | 3 | 3 | 50 | 0.001487 | nothing collected | 2.93 | 1.93 | 1.5 |
| 3 | 3 | 2.35 | 50 | 0.001899 | 0.26 | 2.3 | 2.0 | 1.1 |

| | | | | | | | | |
|---|---|---|---|---|---|---|---|---|
| Reactor temperature = 45° C Reactor pressure = 4 MPa [Cr] = 0.016 mmol/litre Al/Cr (molar) = 300/1 Ligand/Cr (molar) = 1/1 | | | | | | | | |

### INDUSTRIAL APPLICABILITY

Many processes for the preparation of alpha olefins by the oligomerization of ethylene also produce some polyethylene as an undesirable contaminant/by product. The process of this invention mitigates the formation of polyethylene during an ethylene oligomerization process. The alpha olefins that are produced according to this invention have a wide variety of commercial uses, particularly as comonomers for the preparation of ethylene copolymers.

## Claims

1. A continuous process for the oligomerization of ethylene in a liquid full, continuously stirred tank reactor, comprising the steps of:
a) feeding ethylene, hydrogen and an oligomerization catalyst system to said reactor, wherein said ethylene and said hydrogen are contacted prior to being added to said reactor and are jointly added to said reactor via a common feed tube;
b) oligomerizing said ethylene in the presence of said catalyst system and said hydrogen;
**characterized in that** said catalyst system contains:
1) a catalyst comprising:
1.1) a source of chromium, and
1.2) a ligand defined by the formula: 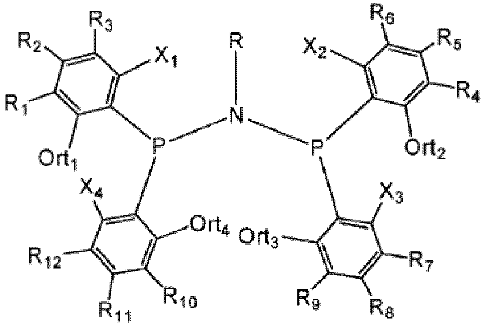 wherein each of X₁ to X₄ is independently selected from the group consisting of F, Br, H, Cl, a polar group and a hydrocarbyl group having from 1 to 30 carbon atoms; and wherein each of R; ort¹ to ort⁴ and R₁ to R₁₂ is independently selected from the group consisting of H and non-interfering substituents; and
2) an activator.

2. The process of claim 1 when conducted at a temperature of from 20°C to 80°C.

3. The process of claim 1 when conducted at a pressure of from 2 to 10 MPa.

4. The process of claim 1 wherein said hydrogen is fed to said reactor in an amount of from 200 to 20,000 parts per million by weight, based on the weight of said ethylene.

5. The process of claim 1 wherein said ligand is (ortho-F-C₆H₄)₂PN (i-Pr)P(ortho-F-C₆H₄)₂.

6. The process of claim 5 wherein said activator comprises an aluminoxane.

7. The process of claim 1 wherein said liquid is a solvent for said catalyst system.

8. The process of claim 7 wherein said solvent is an alphatic hydrocarbon.

9. The process of claim 1 wherein said liquid is at least one ethylene oligomer selected from the group consisting of hexenes, octenes and mixtures thereof.

## Patentansprüche

1. Kontinuierliches Verfahren zur Oligomerisation von Ethylen in einem mit Flüssigkeit gefüllten kontinuierlichen Rührkesselreaktor, wobei das Verfahren die folgenden Schritte umfasst:
a) das Einspeisen von Ethylen, Wasserstoff und eines Oligomerisationskatalysatorsystems in den Reaktor, wobei das Ethylen und der Wasserstoff vor der Zugabe zu dem Reaktor in Kontakt gebracht werden und über ein gemeinsames Zufuhrrohr zusammen dem Reaktor zugeführt werden;
b) das Oligomerisieren des Ethylens in Gegenwart des Katalysatorsystems und des Wasserstoffs;
**dadurch gekennzeichnet, dass** das Katalysatorsystem Folgendes enthält:
1) einen Katalysator, der Folgendes umfasst:
1.1) eine Chromquelle und
1.2) einen durch die folgende Formel definierten Liganden: 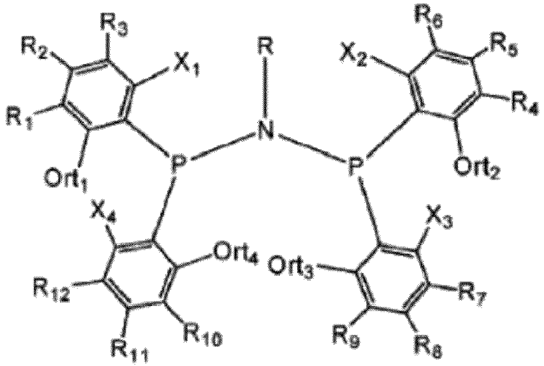 worin die X₁ bis X₄ jeweils unabhängig aus der aus F, Br, H, Cl, einer polaren Gruppe und einer Hydrocarbylgruppe mit 1 bis 30 Kohlenstoffatomen bestehenden Gruppe ausgewählt sind; und worin die R, Ort₁ bis Ort₄ und R₁ bis R₁₂ jeweils unabhängig aus der aus H und nichtstörenden Substituenten bestehenden Gruppe ausgewählt sind; und
2) einen Aktivator.

2. Verfahren nach Anspruch 1, durchgeführt bei einer Temperatur von 20 °C bis 80 °C.

3. Verfahren nach Anspruch 1, durchgeführt bei einem Druck von 2 bis 10 MPa.

4. Verfahren nach Anspruch 1, wobei der Wasserstoff, bezogen auf das Gewicht des Ethylens, in einer Menge von 200 bis 20.000 Gew.-ppm in den Reaktor eingespeist wird.

5. Verfahren nach Anspruch 1, wobei der Ligand (ortho-F-C₆H₄)₂PN(i-PR)P-(ortho-F-C₆H₄)₂ ist.

6. Verfahren nach Anspruch 5, wobei der Aktivator ein Aluminoxan umfasst.

7. Verfahren nach Anspruch 1, wobei die Flüssigkeit ein Lösungsmittel für das Katalysatorsystem ist.

8. Verfahren nach Anspruch 7, wobei das Lösungsmittel ein aliphatischer Kohlenwasserstoff ist.

9. Verfahren nach Anspruch 1, wobei die Flüssigkeit zumindest ein Ethylenoligomer ist, das aus der aus Hexenen, Octenen und Gemischen davon bestehenden Gruppe ausgewählt ist.

## Revendications

1. Procédé en continu pour l'oligomérisation d'éthylène dans un réacteur à réservoir rempli de liquide et continuellement agité, comprenant les étapes consistant à :
a) alimenter de l'éthylène, de l'hydrogène et un système de catalyseur d'oligomérisation dans ledit réacteur, dans lequel ledit éthylène et ledit hydrogène sont mis en contact avant d'être ajoutés audit réacteur et sont ajoutés conjointement audit réacteur via un tube d'alimentation commun ;
b) oligomériser ledit éthylène en présence dudit système de catalyseur et dudit hydrogène ;
**caractérisé en ce que** ledit système de catalyseur continent :
1) un catalyseur comprenant :
1.1) une source de chrome, et
1.2) un ligand défini par la formule : 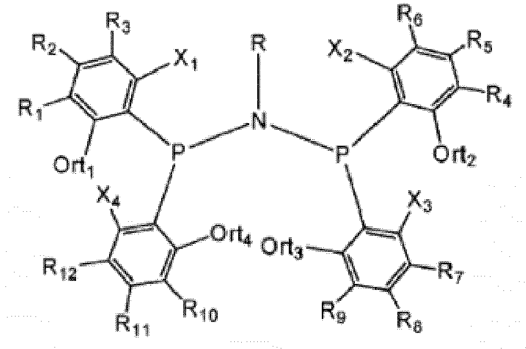 dans lequel chacun de X₁ à X₄ est sélectionné indépendamment dans le groupe constitué par F, Br, H, CL, un groupe polaire et un groupe hydrocarbyle ayant de 1 à 30 atomes de carbone ; et dans lequel chacun de R ; ort¹ à ort⁴ et R₁ à R₁₂ est sélectionné indépendamment dans le groupe constitué de H et de substituants non interférants ; et
2) un activateur.

2. Procédé selon la revendication 1, lorsqu'il est mis en oeuvre à une température de 20°C à 80°C.

3. Procédé selon la revendication 1, lorsqu'il est mis en oeuvre à une pression de 2 à 10 MPa.

4. Procédé selon la revendication 1, dans lequel ledit hydrogène est alimenté dans ledit réacteur en une quantité de 200 à 20 000 parties par million en poids, par rapport au poids dudit éthylène.

5. Procédé selon la revendication 1, dans lequel ledit ligand est (ortho-F-C₆H₄)2PN(i-Pr)P(ortho-F-C₆H₄)₂.

6. Procédé selon la revendication 5, dans lequel ledit activateur comprend un aluminoxane.

7. Procédé selon la revendication 1, dans lequel ledit liquide est un solvant pour ledit système de catalyseur.

8. Procédé selon la revendication 7, dans lequel ledit solvant est un hydrocarbure aliphatique.

9. Procédé selon la revendication 1, dans lequel ledit liquide est au moins un oligomère d'éthylène sélectionné dans le groupe constitué par des hexènes, des octènes et des mélanges de ceux-ci.
